# EUROPEAN PATENT APPLICATION

(11) **EP 1 167 527 A1**
(43) Date of publication of application: **02.01.2002**
(21) Application number: 00870140.1
(22) Date of filing: 22.06.2000
(51) Int. Cl.: C12N 15/19, C07K 14/52, C07K 16/24, A61K 38/19, G01N 33/68, C12N 9/72, A61K 31/70, A61K 38/49, C12Q 1/68

(54) **Processed human chemokines PHC-1 and PHC-2**

(71) Applicant: Euroscreen S.A., 1070 Brussels (BE); Niedersächsisches Institut für Peptid-Forschung GmbH (IPF), 30625 Hannover (DE)
(72) Inventor: Detheux, Michel, 7000 Mons (BE); Parmentier, Marc, 1070 Brussels (BE); Kirchhoff, Frank, 91054 Erlangen (DE); Ständker, Ludger, 30625 Hannover (DE); Forssmann, Wolf-Georg, 30625 Hannover (DE)
(74) Representative: Van Malderen, Joelle

(57) **Abstract**

The present invention is related to newly identified compounds, polynucleotide sequences encoding the amino acid sequences of said compounds, as well as agonists, antagonists or inhibitors of said compounds for chemokine receptors, especially the CCR-5 receptor and their use in the field of diagnostic and therapeutics involving said chemokine receptors.

## Description

### Field of the invention

The present invention relates to newly identified compounds, polynucleotide sequences encoding the amino acid sequences of said compounds, as well as antagonists or inhibitors derived from said compounds for chemokine receptors and their use in the field of diagnostic and therapeutics involving said chemokine receptors.

### State of the art

Chemokines are small sequences which are known to play fundamental role in the physiology of acute and chronic inflammatory processes, as well as in the pathological deregulation of these processes.

Furthermore, several chemokine receptors, especially the chemokine receptors identified as CCR5, CXCR4, and CCR3 are known to be involved in HIV viral infection of a patient.

The known chemokines MIP-1α, MIP-1β, RANTES MCP-2 and synthetic compounds derived from these chemokines (AOP-RANTES) were described as major HIV inhibitory factors.

Furthermore, an intensive research has been made upon the screening of new compounds (generally synthetic derivatives of natural chemokines and synthetic chemical compounds obtained from library screening) having improved characteristics as viruses antagonists or agonists to chemokine receptors.

### Aims of the invention

The present invention aims to provide new natural compounds, as well as synthetic or natural derivatives thereof, which are antagonists or inhibitors to various chemokine receptors and HIV-1 and HIV-2 co-receptors, especially to the CCR-1 and CCR-5 receptor, and which find application in the treatment and/or the prevention of various diseases.

### Summary of the invention

The present invention is related to a new compound, preferably a chemokine compound which presents more than 60%, preferably more than 70%, more than 75%, more than 80%, more than 85%, more than 90% or more than 95% homology (or sequence identity) with SEQ ID NO: 1 (GPYHPSECCFTYTTYKIPRQRIMDYYETNSQCSKPGIVFITKRGHSVCTNPSDKWVQD YIKDMKEN) or SEQ ID NO: 2 (HPSECCFTYTTYKIPRQRIMDYYETNSQCSKPGIVFITKRGHSVCTNPSDKWVQDYIK DMKEN), with the proviso that said compound does not correspond to the amino acid sequences SEQ ID NO: 3 (MKISVAAIPFFLLITIALGTKTESSSRGPYHPSECCFTYTTYKI PRQRIMDYYETNS QCSKPGIVFITKRGHSVCTNPSDKWVQDYIKDMKEN) or SEQ ID NO: 4 (TKTESSSRGPYHPSECCFTYTTYKI PRQRIMDYYETNSQCSKPGIVFITKRGHSVCT NPSDKWVQDYIKDMKEN) (HCC-1 peptide), which is an untruncated PHC-1 polypeptide already described in the documents P4344397.4 and P4427395.9 and by P. Schulz-Knappe et al. (J. Exp. Med., Vol. 183, pp. 295-299 (1999)).

The new compound is preferably an antagonist of the HIV-viruses to chemokine receptors, especially to the CCR-1, CCR-3 and CCR-5 chemokine receptors.

Preferably, the polypeptide according to the invention is the chemokine PHC-1 having the amino acid sequence SEQ ID NO: 1, SEQ ID NO: 2 or biologically active fragments or portions thereof.

Said active fragments or portions thereof are advantageously NH₂-terminal amino acid sequences of at least 10, 15, 20, 25, 30 amino acids, preferably at least 40 amino acids, more preferably at least 50 or 55 amino acids, of the original above-described complete sequences SEQ ID NO: 1 or SEQ ID NO: 2, which may include the deletion of one or more amino acids; as well as their derivatives, in particular compounds having at least 10, 15, 20, 25, 30, 40, 50 or 55 amino acids of the complete amino acid sequences SEQ ID NO: 1 or SEQ ID NO: 2 with one or more additional amino acid residue(s) in the sequence, possibly linked to or modified by (substitution of one or more carbon atoms or one or more alkyl) amide, acetyl, phosphoryl and/or glycosyl or other substitution groups.

The modification of the original amino acid sequences SEQ ID NO: 1 or SEQ ID NO: 2 is preferably obtained upon the C-terminal end by fusion with other amino acid sequences Tags, or the incorporation of the above-identified groups including fluorescent groups upon one or both extremities of the original sequence SEQ ID NO: 1 in order to provide a substrate for the proteolytic activity screening. Among said polypeptide and their derivatives (analogues) are excluded the compounds having the amino acid sequence SEQ ID NO: 3 or SEQ ID NO: 4 and their active amidated, acetylated, phosphorylated and/or glycosylated derivatives, which is the compound which does not present the activity of the compound according to the invention, which is not able to bind to the following chemokine receptors CCR-1, CCR-3, CXR-4 and/or CCR-5.

Preferably, additional amino acid residues upon the N-terminal or C-terminal portion of the sequence SEQ ID NO: 1 or SEQ ID NO: 2 are chains of 2 to 10 amino acids.

According to a preferred embodiment of the present invention, the derivatives or analogues of the compound according to the invention are amino acid sequence SEQ ID NO: 1 or SEQ ID NO: 2 comprising a coupling with a chemical group upon the N-terminal end.

In the following description, the peptides according to the invention are identified as PHC chemokines or PHC derivatives or analogues, which correspond to PHC peptides wherein a portion, preferably the N-terminus extremity, is modified by its coupling or its substitution with a chemical group, preferably according to the method described by H. Gaertner et al. (J. Biol. Chem., Vol. 271, pp. 2591-2603 (1996) and G. Simmons et al. (Science, Vol. 276, pp. 276-279 (1997)).

Preferably, said PHC derivatives or analogues have one of the following structure:
- [Glyoxyloyl¹]PHC 1-Pentane oxime,
- [Glyoxyloyl¹]PHC Caproyl oxime,
- D-Met-[Gly¹]PHC,
- L-Pyrollidone Carboxoyl-[Gly¹]PHC,
- [Glyoxyloyl¹]PHC,
- [Glyoxyloyl¹]PHC 1-acetyl-ethylamine-2oxime,
- [Glyoxyloyl¹]PHC S-Methyl 1-Thiopropane-3-oxime,
- [Glyoxyloyl¹]PHC 2-Pentene oxime,
- [Glyoxyloyl¹]PHC Methane oxime,
- Hexanoyl-[Gly¹]PHC,
- [Glyoxyloyl¹]PHC Phenylmethane oxime,
- [Glyoxyloyl¹]PHC 1-Propane oxime,
- [Glyoxyloyl¹]PHC 1-Butane oxime,
- [Glyoxyloyl¹]PHC 2-Butane oxime,
- Hexanyl-[Gly¹]PHC,
- [Glyoxyloyl¹]PHC 2-Propane oxime,
- [Glyoxyloyl¹]PHC Pentane oxime,
- Nonanyl-PHC,
- [Glyoxyloyl¹]PHCPHC 2-Heptane oxime,
- [Glyoxyloyl¹]PHC Ethane oxime,
- [Glyoxyloyl¹]PHC 1-Heptane oxime,
- [Glyoxyloyl¹]PHC 1-hexane oxime,
- [Glyoxyloyl¹]PHC 1-Pentene oxime,
- Nonanoyl-PHC,
or are a compound having one of the following formulas:

- CH₃-(CH₂)₄-CO-NH-CH₂-CO-PHC,

- CH₃-(CH₂)₅-H-CH₂-CO-PHC,

- CH₃-(CH₂)₇)-CO-PHC,

- CH₃-(CH₂)₈)-PHC,

- HOOC-(CH₂)₅-O-N=CH-CO-PHC.

Analogues of said compound are also molecules such as antibodies or other products obtained by recombinant chemistry or library screening which may mimic and preferably increase the interactions of said compounds to their receptors.

According to another preferred embodiment of the present invention, one or more amino acids, preferably lysine, histidine, glutamate, aspartate, or cysteine residue of the PHC peptides are modified by a coupling with a chemical group having the structure of a polyethyleneglycol. Such modification allows an increasing of the plasma half-life time of the original PHC molecule.

Furthermore, the compound according to the invention for diagnostic purpose comprises a label upon one or more of its extremities, preferably a label selected from the group consisting of radioactive labels, biotin or streptavidin molecules, enzymes and/or fluorescent labels.

The preferred PHC chemokine compounds according to the invention are of human origin and can be obtained by an isolation procedure departing from human blood ultrafiltrate (hemofiltrate) and by using biological assay systems determining their biological activity.

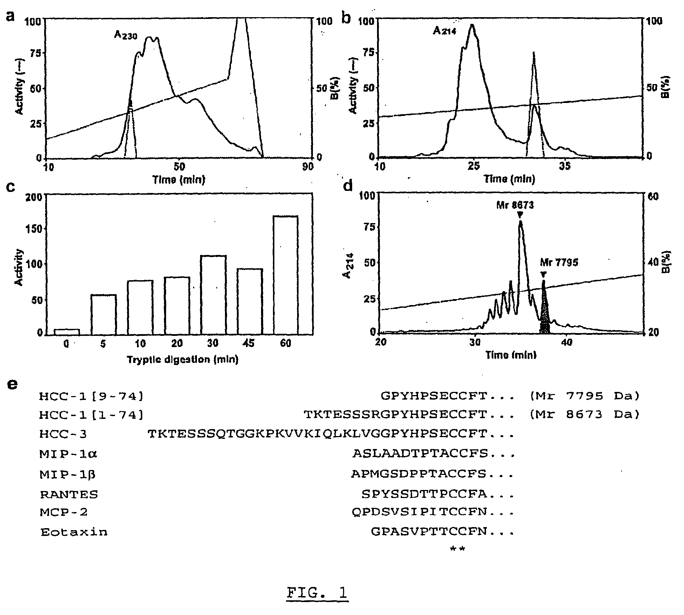

As shown in Fig. 1 and in order to achieve the purification of the compound according to the invention, peptides are prepared from human hemofiltrate as described by Schulz-Knaap et al., J. Chrom. A., Vol. 776, pp. 125-132 (1997). Thereafter, the obtained hemofiltrate fractions were screened for their chemokine receptor(s) stimulatory activity, after what the biologically active fractions were further purified by chromatography procedures using diverse reverse phase column chromatographic steps as described in the Example 1.

The biologically active peptides obtained by the chromatographic purification were subjected to a structure determination, including mass spectrometry, and peptide sequence analysis.

However, such compounds could also be obtained by recombinant genetic technologies or by synthesis, said methods possibly comprising also purification steps that can be carried out by the person skilled in the art.

A second aspect of the present invention is related to a polynucleotide comprising at least a sequence encoding the compound according to the invention, preferably a polynucleotide sequence encoding the compound or its active portions having the amino acid sequence SEQ ID NO: 1 or SEQ ID NO: 2 or biologically active fragments or portions thereof presenting the activity of the full sequence.

Another aspect of the present invention is related to not known antagonists or inhibitors of the compounds of the invention or the polynucleotide encoding the amino acid sequence of said compounds to a chemokine receptor, preferably a receptor selected from the group consisting of the CCR-1, CCR-3 and CCR-5 receptors, preferably to the CCR-5 receptor.

An antagonist of the compound according to the invention means a molecule or a group of molecules able to bind to the receptor and block the binding of the compound according to the invention to said receptor. Said unknown antagonist is also an antagonist to known "natural" compound, including micro-organisms such as bacteria, protozea, viruses or portions thereof (binding to said chemokine receptor), in particular immunodeficiency viruses 1 and/or 2 (HIV-1 and/or HIV-2) or other viruses affecting a patient. Preferably, said viruses are selected from the group consisting of herpes simplex virus, varicella zoster virus, hepatitis-A viruses, hepatitis-B viruses, zytomegalo virus, influenza virus, polio virus, rhino virus, measles virus, German measles virus, rabies virus, Rous sarcoma virus, Pox virus and Epstein-Barr virus.

Said known "natural" compound could be also specific portions of said viruses able to bind to said chemokine receptor. Examples of such portions are the GP 120/160 glycoprotein of HIV-1 and/or HIV-2 which is known to interact specifically with the CCR-5 receptor.

An inhibitor of the compound according to the invention is a molecule directed against said compound or a nucleotide sequence encoding said compound, in order to possibly block its binding and interactions to other molecules, including the receptor according to the invention.

Examples of said inhibitor are (possibly labelled) (monoclonal or polyclonal) antibodies or hypervariable portions of said antibodies. Said antibody (monoclonal or poyclonal) or its hypervariable portion (Fab', Fab2', etc.) as well as the hybridoma cell producing said antibody are a further aspect of the present invention, which finds specific industrial application in the field of diagnostic, especially for the monitoring of the effect of the compound according to the invention upon chemokine receptors, especially CCR-1, CCR-3 and/or CCR-5 receptors.

As mentioned above, the present invention is also related to the polynucleotide encoding the amino acid sequence of the compound according to the invention (such as a cDNA molecule, genomic DNA molecule or RNA molecule).

Another aspect of the present invention is related to a vector comprising said polynucleotide, preferably a vector adapted for expression in a cell, and which comprises the regulatory element necessary for expression of said polynucleotide in a cell (preferably a cell selected from the group consisting of a bacterial cell, a yeast cell, an insect cell or a mammalian cell).

Said vector could be a plasmid or a virus, preferably a baculovirus, an adenovirus, a retrovirus or a semliki forest virus.

Another aspect of the present invention is related to the cell transformed (according to known techniques by the person skilled in the art) by the vector according to the invention, preferably a mammalian cell, (such as a cell selected from the group consisting of COS-7 cell, CHO-K1 cell, LM(tk-)cell, NIH-3T3 cell, HEK-293 cell or K-562 cell.

The inhibitor of the compound according to the invention could be also an nucleic acid probe of more than 15, 20, 25 or 30 nucleotides, such as an antisense oligonucleotide having a sequence capable of specifically hybridising to a mRNA molecule encoding the compound according to the invention, so as to prevent translation of said mRNA molecule or an antisense oligonucleotide or a ribozyme capable of specifically hybridising to a DNA molecule encoding the compound according to the invention, said antisense oligonucleotide comprising possibly chemical analogues of nucleotides.

The present invention is also related to a transgenic non human mammal comprising an homologous recombination "knock-out" of the polynucleotide according to the invention or a transgenic non human mammal overexpressing above natural level the polynucleotide and the compound according to the invention. Such transgenic non-human mammal can be obtained by methods well known by the person skilled in the art, for instance the one described in the document WO98/20112 using the classical techniques based upon the transfection of embryonic stem cells preferably according to the method described by Carmeliet et al. (Nature, Vol. 380, pp. 435-439 (1996)).

Preferably, said transgenic non human mammal overexpressing the polynucleotide according to the invention comprises said polynucleotide incorporated in a DNA construct with a inducible promoter allowing the overexpression of the compound according to the invention.

Possibly, said nucleic acid construct comprises also tissue and specific regulatory elements.

Another aspect of the present invention is related to a method for the screening (detection and possibly recovering) of molecules which are not known to be agonists, antagonists or inhibitors of the new compound according to the invention to a chemokine receptor, more preferably to the CCR-5 chemokine receptor, said molecules being not known "natural" molecules binding to said chemokine receptor(s), especially MIP-1α, MCP-2, MIP-1β or RANTES chemokines to the CCR-5 chemokine receptor.

Said method comprises the steps of:
- contacting a cell or cell extract from the cells transfected with a vector expressing a polynucleotide encoding said receptor,
- possibly isolating a membrane fraction from the cell extract or the complete cell with the compound according to the invention under conditions permitting binding of said compound to said receptor, possibly by the activation of a functional response, and
- detecting the presence of any such compound by means of a bio-assay (such as a modification, the production of a second messenger or an increasing in the receptor activity) in the presence of the molecule working as an agonist, antagonist or inhibitor to the compound, thereby (possibly recovering and) determining whether said molecule is able to work as an agonist, antagonist or inhibitor of the compound according to the invention to its receptor.

Preferably, the second messenger assay comprises the measurement of intracellular cAMP, intracellular inositol phosphate, intracellular diacylglycerol concentration, arachinodic acid con, MAP kinases or thyrosine kinases pathways or intracellular calcium mobilisation.

Another aspect of the present invention is related to a pharmaceutical composition (vaccine), comprising an effective amount of one or more compounds according to the invention, their analogues, antagonists or inhibitors (directed against said compounds), as well as the polynucleotides encoding said compounds and a pharmaceutically adequate carrier or diluent for use as a medicament.

Advantageously, another aspect is related to the use of one or more of said compounds, analogues, antagonists or inhibitors and polynucleotides for the manufacture of a medicament in the treatment and/or the prevention of various diseases (wherein the CCR-1, CCR-3 and CCR-5 are involved), especially viral infections, in particular diseases (AIDS) induced by a human immunodeficiency virus 1 and/or 2 (HIV-1 and/or HIV-2) or the other viruses above described; as well as for the preparation of a medicament for the treatment and/or the prevention of disturbances of cell migration, diseases or perturbations of the immune system, including cancers, development of tumours and tumour metastasis and Hodgkins lymphoma, rheumatoid arthritis, psoriasis, chronic contact dermatitis, inflammatory bowel disease, multiple sclerosis (MS), stroke, sarcoidosis, organ transplant rejection, inflammatory and neoplastic processes, viral, bacterial and fungal infections, for wound and bone healing and dysfunctions of regulatory growth functions, pain, diabetes, obesity, anorexia, bulimia, Parkinson's disease, acute heart failure, hypotension, hypertension, urinary retention, osteoporosis, angina pectoris, myocardial infarction, restenosis, atherosclerosis, diseases characterised by excessive smooth muscle cell proliferation, aneurysms, wound healing, diseases characterised by loss of smooth muscle cells or reduced smooth muscle cell proliferation, stroke, ischemia, ulcers, allergies (including asthma), benign prostatic hypertrophy, migraine, vomiting, psychotic and neurological disorders, including anxiety, schizophrenia, maniac depression, depression, delirium, dementia and severe mental retardation, degenerative diseases, neurodegenerative diseases such as Alzheimer's disease, and dyskinasias such as Huntington's disease or Gilles de la Tourett's syndrome among others.

Another aspect of the present invention is related to a diagnostic kit or device comprising the (possibly labelled) compounds, analogues, antagonists and/or inhibitors and polynucleotides according to the invention.

A preferred embodiment of said diagnostic kit or device is a kit or device for the monitoring of the activity of said compounds upon various chemokine receptors, especially the monitoring of their activity with a correlation to their therapeutic or prophylactic action upon one or more of the above mentioned diseases or symptoms of said diseases.

According to another preferred embodiment of the present invention, said diagnostic kit or device is a high throughput screening diagnostic and/or dosage device, intended for high throughput identification, recovering and/or dosage of such compounds, analogues, antagonists or inhibitors which allow the identification of high amounts of compounds (known or unknown), acting as antagonists or inhibitors of the compounds according to the invention.

Preferably, said high throughput screening diagnostic and/or dosage device is based upon the method described in the international patent application WO 00/02045.

Said high throughput screening technology could be performed upon various solid supports, such as microtiter plates or biochips according to known techniques to the person skilled in the art.

The present invention is also related to the molecule characterised and possibly recovered by said method, to the molecule identified by said method and to a pharmaceutical composition (vaccine) comprising said molecule and a pharmaceutically acceptable carrier or diluent.

Another aspect of the present invention is related to a molecule, possibly a recombinant molecule, preferably a protease including recombinant protease, such as an urokinase or staphylokinase, responsible for the generation of the PHC-1 and PHC-2 compounds (identified as SEQ ID NO: 1 and SEQ ID NO: 2 generated naturally or artificially from SEQ ID NO: 3 or SEQ ID NO: 4), as well as inhibitors or activators of said molecule for providing advantageously a treatment or a prevention of the associated diseases, especially the ones above described. Such molecules, including recombinant molecules having possibly increased enzymatic activity (obtained and defined by known screening techniques), are advantageously administered according to known techniques by the person skilled in the art to a patient in order to obtain the cleavage of the molecule SEQ ID NO: 3 or SEQ ID NO: 4 into the specific PHC-1 and PHC-2 compounds according to the invention, and possibly their modification into improved derivatives. The method of treatment of the above-identified diseases or the symptoms associated with the above-identified diseases is obtained by using a sufficient amount of said molecules, preferably said proteases, which will be administered to a patient which may suffer from the above-identified diseases or symptoms, in order to allow or increase (facilitate) the cleavage of the compound corresponding to SEQ ID NO: 3 or SEQ ID NO: 4 into the compounds according to the invention.

Preferably, the pharmaceutical composition according to the invention is also a nucleotide sequence encoding the above-identified recombinant proteases for an *in vivo* treatment or for a treatment by a cell for an *ex vivo* treatment. Said cell will be readministered to a patient to obtain a genetic therapy or prophylaxis of a patient suffering from said diseases.

Another aspect of the present invention is related to the use of the recombinant molecules according to the invention for the preparation of a medicament intended for the prevention and/or the treatment of the above-identified diseases or symptoms of said diseases, including the use of a nucleotide sequence encoding said recombinant molecules, and being possibly included into a vector or expressed in a cell that will be administered either in *vivo* or *ex vivo* by techniques known by the person skilled in the art.

The present invention is also related to the use of variants of said molecules and proteases in order to improve the prevention and/or therapeutic method according to the invention or for reducing the possible side-effects induced by said method upon the patient. Such modification may include the deletion or the addition of one or more nucleotides, amino acids or chemical group to said molecules or nucleotide sequence encoding said molecules.

The present invention will be described hereafter in reference to the enclosed figures.

### Short description of the drawings

Figure 1 is representing the purification of the compound PHC-1 from human hemofiltrate.

Figure 2 is representing the binding of functional activity of the pH compound on human recombinant receptors expressed in CHO-K1 cells.

Figure 3 is representing the calcium mobilisation and the chemotaxis induced by PHC-1 compound in primary cell populations.

Figure 4 is representing the inhibition of HIV-1 infection.

Figure 5 is representing the screening of human cell lines for PHC-1 proteolytic activation

Figure 6 is representing the inhibition of proteolytic generation of PHC-1 by various serine protease inhibitors.

### Detailed description of the invention

Surprisingly, it has been shown that specifically processed chemokines PHC-1 and PHC-2 can be isolated from human hemofiltrate and represent highly active agonist of different chemokine receptors. The processed chemokine PHC-2 is an N-terminal truncated form of PHC-1 and both polypeptides occur in human hemofiltrate and could represent the naturally processed and biologically highly active forms of the known chemokine precursor sequence of the chemokines HCC-1 (Schulz-Knappe et al. 1996, Journ. Exp. Med. 183, pp. 295-299; accession No. Q16627) or HCC-3 (accession No. Q13954).

The newly identified chemokines PHC-1 and PHC-2 belong to the family of beta-chemokines. These beta-chemokines have numerous functions related to inflammation or wound healing, immunoregulation, cancer, infectious diseases and to a number of further disease conditions.

The peptide PHC-1 according to the invention shows a very high binding affinity and a very potent stimulatory activity to the chemokine receptors CCR1, CCR3, and CCR5. No effects of PHC-1 on the chemokine receptors, CCR2, CCR4, CCR6, CCR7, CCR8, CCR9, CXCR1 and CXCR4 is observed. The peptide PHC-1 according to the invention affect chemotaxis/migration of eosinophils, T-lymphocytes, monocytes and dendritic cells and may be therefore involved in inflammatory conditions in humans or may be used as therapeutic and/or diagnostic agent in inflammatory, immunological, cancer diseases and infections such viral, bacterial, fungal and protozoan infections, particularly infections caused by HIV-1 or HIV-2.

Due to the very high binding affinity to chemokine receptor CCR5, which represents the most important HIV-1 coreceptor, the PHC-1 polypeptide affects a very potent inhibition of HIV infection and HIV replication in human cells.

The processed chemokines PHC-1 and PHC-2 are of human origin and can be obtained by an isolation procedure departing from human blood ultrafiltrate (hemofiltrate) and by using biological assay systems determining their biological activity. To achieve the purification of PHC-1 and PHC-2, peptides are prepared from human hemofiltrate as described recently in the literature (Schulz-Knappe et al.1997, J. Chrom. A, 776, 125-132). The obtained hemofiltrate fractions were screened for their chemokine receptor-stimulatory activity. Then the biologically active fractions have to be further purified by chromatographic procedures using diverse reverse phase column chromatographic steps.

The biologically active peptides obtained by chromatographical purification were subjected to a structure determination including mass spectrometry and peptide sequence analysis.

In addition to the recombinant production of PHC-1 and PHC-2, a stepwise total synthesis on usual solid phases in terms of Merrifield synthesis is also possible.

### Examples

### Example 1: Isolation of human chemokines PHC-1 and PHC-2 from hemofiltrate

Peptides of human hemofiltrate were prepared as described recently in the literature (Schulz-Knappe, P. et al.1997, J. Chrom. A, 776, 125-132). In brief, the peptides were extracted from 10,000 L of human hemofiltrate obtained from a local nephrological center. The collected hemofiltrate had been derived from 40 adult patients with chronic renal disease. Immediately after blood filtration using ultrafilters with a specified cut-off of 20 kDa, the filtrate was routinely chilled to 4°C and adjusted to pH 3 to prevent bacterial growth and proteolysis. After dilution with deionized water to a conductivity of < 8 mS/cm, the batches of 800-1000 L hemofiltrate were conditioned exactly to pH 2.7 using hydrochloric acid. These batches were applied to a strong cation-exchanger (2 l Fractogel TSK SP 650(M), Merck) and the peptides were batch-eluted with 10 1 0.5 M ammonium acetate, pH 7.0. These eluates were stored at -20°C. To eliminate remaining amounts of plasma albumine in the concentrated hemofiltrate an additional ultrafiltration step was carried out with pooled eluates corresponding to 10.000 L equivalent of hemofiltrate. Ultrafiltration was performed by a sartocon-mini (0.1 m², ps-membrane) ultrafilter with a specified Mr cut-off of 30 kDa. Filtration was driven by a transmembranous pressure gradient of 1 bar at a temperature of 5°C and a flow rate of 5-6 L/h.

For the first purification step of PHC-1 and PHC-2 the ultrafiltrate was diluted with deionized water to a conductivity of 6.7 mS / cm, adjusted to pH 2.7 with HCl, and applied to a second 10 l Fractogel cation-exchange column. The column was washed with 0.01 M HCl until conductivity was below 1 mS / cm. Stepwise batch elution of the bound peptides was performed using the eight following buffers: I: 0.1 M citric acid pH 3.6; II: 0.1 M acetic acid + 0.1 M sodium acetate, pH 4.5; III: 0.1 M malic acid, pH 5.0; IV; 0.1 succinic acid, pH 5.6; V: 0.1 M sodium dihydrogenphosphate, pH 6.6; VI: 0.1 M disodium hydrogenphosphate, pH 7.4; VII: 0.1 M ammonium carbonate, pH 9.0; VIII: water, pH 7.0 (desalting step). The resulting pH pool fractions (15 to 25 L) were collected, acidified to pH 2-3, and immediately subjected to the second separation step.

For the second purification step of PHC-1 and PHC-2 the eluate of pH pool fraction No. 5 was loaded onto a Source RPC column (15 *µ*m, 10 x 12.5 cm, Pharmacia), washed with two column volumes of solvent A (10 mM HCl) and separation was performed at a flow rate of 200 ml / min by a 8 1 gradient from 100 % A (water, 10 mM HCl) to 60 % B (80 % acetonitrile (v/v), 10 mM HCl). Fractions of 200 ml were collected and the absorbance at 280 nm was monitored. Aliquots of these peptide fractions were lyophilized and tested for bioactivity as described below.

Fraction 21 contained the biologically active peptides according to this invention (Figure 1A).

For the third purification step of PHC-1 and PHC-2 the bioactive fraction 21 was loaded on a RP-C18 column (15-20 *µ*m, 300 Å, 47 x 300 mm; Vydac, Hesperia, USA) and separation was performed under a flow rate of 40 ml/min using following gradient and buffers: from 90 % A (water, 10 mM HCl) to 50 % B (80% acetonitrile, 10 mM HCl) in 48 min. Single fractions of 1 min were collected, monitoring the absorbance at 214 nm, and tested for bioactivity as described below. Fraction 12 contained the biologically active peptides according to this invention.

For the fourth purification step of PHC-1 and PHC-2 the bioactive fraction 12 was loaded on a RP-C4 column (5 *µ*m, 100 Å, 20 x 250 mm; Biotek Silica, Östringen, Germany) and separation was performed under a flow rate of 7 ml/min using following gradient and buffers: from 67 % A (water, 0.1% trifluoroacetic acid) to 58 % B (80% acetonitrile, 0.1% trifluoroacetic acid) in 60 min. Single fractions of 1 min were collected, monitoring the absorbance at 214 nm, and tested for bioactivity as described below. Fractions 13+14 contained the biologically active peptides according to this invention.

For the fifth purification step of PHC-1 and PHC-2 the obtained bioactive fractions 13 + 14 were further seperated on analytical RP-C18 column (5 µm, 30 nm, 1.0 x 25 cm; Vydac; flow rate: 1.8 ml/min) using following gradient and buffers: from 70 % A (water, 0.1% trifluoroacetic acid) to 45 % B (80% acetonitrile, 0.1% trifluoroacetic acid) in 45 min. Single fractions of 1 min were collected, monitoring the absorbance at 214 nm, and tested for bioactivity as described below. Fractions 19 + 20 contained the biologically active peptides according to this invention.

For the sixth purification step of PHC-1 and PHC-2 the obtained bioactive fractions 19 + 20 were loaded onto an analytical RP-C18 column (5 µm, 30 nm, 0.46 x 25 cm; YMC, Schernbeck, Germany; flow rate: 0.6 ml/min) using following gradient and buffers: from 77 % A (water, 0.1% trifluoroacetic acid) to 38 % B (80% acetonitrile, 0.1% trifluoroacetic acid) in 45 min. Single fractions of 1 min were collected, monitoring the absorbance at 214 nm, and tested for bioactivity as described below. Fraction 20 contained the biologically active peptides according to this invention.

For the seventh purification step of PHC-1 and PHC-2 the obtained bioactive fraction 20 was loaded onto an analytical RP-C18AQ column (3 µm, 0.1 x 25 cm; Reprosil-pur, Fa. Maisch, Ammerbuch 3, Germany; flow rate: 0.02 ml/min) using following gradient and buffers: from 77 % A (water, 0.1% trifluoroacetic acid) to 38 % B (80% acetonitrile, 0.1% trifluoroacetic acid) in 45 min. Single fractions of 0.5 min were collected, monitoring the absorbance at 214 nm, and tested for bioactivity as described below. The purified, biologically active peptides PHC-1 and PHC-2 were found in the bioactive fractions 45 and 46 (Figure 1B).

A fraction resulting from cation-exchange chromatography was applied to reverse phase-HPLC (C18 column) and the biological activity of the fractions on human CCR5 was assayed (dashed line). Fifth and final purification step of the active material by reverse phase-HPLC (C4 column). The active peak (dashed line) was analyzed by mass spectrometry and Edman degradation, identifying PHC-1 (Mr 7795) and full-size HCC-1 (Mr 8673) as the major compounds in this fraction. Time-course of the CCR5 stimulatory activity generated by tryptic digestion of HCC-1. RP-HPLC chromatogram of trypsin-digested HCC-1 (1 h incubation), showing the generation of bioactive (shaded) PHC-1 (Mr 7795) as one of the main degradation products. Amino acid sequence of the N-terminal part of full-size HCC-1, PHC-1, HCC-3 and other chemokines active on CCR1, CCR3 or CCR5. The asterisks mark the first two conserved cysteines.

### Example 2: Peptide analytics

The biologically active peptides obtained by the seventh chromatographic step (example 1) were subjected to a structure determination. Mass determination of the purified peptides was carried out on a Sciex API III quadrupol mass spectrometer (Sciex, Perkin-Elmer) with an electrospray interface (ESI-MS). The molecular mass of the newly identified peptide PHC-1 was determined to be 7795+/-0.9 Da. The molecular mass of the newly identified peptide PHC-2 was determined to be 7479+/-1 Da (Figure 1E).

The newly identified biologically active peptides were sequenced on an 473 A gas-phase sequencer (Applied Biosystems) by Edman degradation with on-line detection of phenylthiohydantoin-amino acids using the standard protocol recommended by the manufacturer.

The following N-terminal sequence was obtained for PHC-1: GPYHPSEXXFTYTTYKIPRQRIMDYYETNSQ... X: no amino acid signal detectable

The following N-terminal sequence was obtained for PHC-2: HPSEXXFTYTTYKIPRQRIMDYYETNSQ... X: no amino acid signal detectable

A data bank comparison was performed on Swiss-Prot and EMBL and databases. A sequence homology was established and showed sequence identity of both PHC-1 and PHC-2 to the precursor sequence of human chemokine HCC-1 (accession No. Q16627) or human chemokine HCC-3 (accession No. Q13954). PHC-1 and PHC-2 could represent the naturally processed, biologically active forms of human chemokine HCC-1 and/or human chemokine HCC-3.

The molecular mass of PHC-1 (7795 Da) was exactly in accordance to the theoretical mass of a peptide comprising the C-terminal amino acid residues 9-74 of the precursor sequence of human chemokine HCC-1 calculated to be 7795 Da. The molecular mass of PHC-2 (7479 Da) was exactly in accordance to the theoretical mass of a peptide comprising the C-terminal amino acid residues 12-74 of the precursor sequence of human chemokine HCC-1 calculated to be 7479 Da.

The processed chemokine PHC-1 was a strong competitor of 125I-Rantes binding to CCR1 with a Ki of 0.023 ± 0.007 nM (Ki ± SEM). PHC-1 was a strong competitor of 125I-Eotaxin binding to CCR3 with a Ki of 2.7 ± 0.8 nM (Ki ± SEM). PHC-1 was a strong competitor of 125I-MIP 1-alpha binding to CCR5 with a Ki of 0.04 ± 0.01 nM (Ki ± SEM).

### Example 3: Biological assay used for purification of PHC-1 and PHC-2 (Aequorin assay)

To achieve the purification of PHC-1 and PHC-2, a biological assay was used measuring the activation of the chemokine receptor CCR5. Therefore, each of the fractions generated during the chromatographic purification procedure as desribed above was tested on CHO-K1 cells which were stable transfected with the chemokine receptor 5 (CCR5; AC P51681). Activation of the cells via the CCR5 receptor was measured by the following Aequorin assay: Measurement of intracellular calcium increases was performed as described (Stables J et al. 1997, Anal. Biochem. 252, 115-126). The various CHO-K1 cells stably expressing CCR5, mitochondrial apoaequorin and Gα16 were collected from plates with Ca²⁺/Mg²⁺-free phosphate buffer saline (Life Technologies, Gaithersburg, MD) supplemented with 5 mM EDTA, pelleted for 2 min at 1000 x g and resuspended in D-MEM-F12 (Life Technologies, Bethesda) at a density of 5 x 10⁶ cells/ml. The active aequorin was reconstituted by incubation of cells during 4 hours at room temperature with 5 *µ*M coelenterazine h (Molecular Probes, Eugene, OR) in D-MEM-F12 medium containing 0.1 mg/ml bovine serum albumin. Following this incubation, cells were diluted tenfold in the same buffer and stirred during 30 minutes before measurement. 50 *µ*l of cell suspension was then injected on 96-well plates containing the samples to be tested. The integrated light emission was recorded over 30 s with a Microbeta Jet (Wallac) or a Microlumat luminometer (EG&G Berthold). The final results were plotted as percentage of the response obtained with 1 *µ*M ATP. The purified peptide PHC-1 (corresponding to the sequence of the peptide HCC-1, residues 9-74) showed potent activation of the CCR5 transfected cells (EC50 ± SEM: 4.8 ±1.2 nM). In comparison, the known beta-chemokines Rantes and MIP 1-alpha were used as positive controls (EC50 ± SEM of 2.4 ± 0.5 nM or 3.3 ± 0.8nM, respectively). In comparison, the peptides HCC-1, residues 1-74 and HCC-1, residues 6-74 showed no effect on the CCR5 transfected cells.

Results are expressed as Relative Light Units (RLU). Symbols represent the same chemokines as for binding panels. Binding and functional assay results are representative of at least 3 independent experiments.

### Example 4: Binding of PHC-1 to different chemokine receptors (Binding and aequorin assays)

The processed chemokine PHC-1 bound with affinity binding to diverse chemokine receptors.

For determination of its binding properties, CHO-K1 cells were used which were stable transfected with the chemokine receptors CCR1, CCR3, CCR5. Therefore, crude membrane extracts prepared from the CHO-K1 cell lines expressing the various GPCR were used in radioligand binding assays. Competition binding assays were performed in Minisorp tubes (Nunc, Roskilde, Denmark) in a total volume of 100 *µ*l containing 0.1 nM iodinated ligand as tracer, variable concentrations of competitors and defined amount of membranes. Total binding was measured in the absence of competitor and non-specific binding was measured with a 100-fold excess of unlabelled ligand. Samples were incubated for 90 minutes at 25°C then filtered through GF/B filters presoaked in 0.3% polyethylenenimine. Filters were counted in a gamma scintillation counter (Packard). Binding parameters were determined with the PRISM software (Graphpad software, San Diego, CA) using non-linear regression applied to an one site competition model.

The processed chemokine PHC-1 was a strong competitor of 125I-Rantes binding to CCR1 with half-maximal inhibitory concentration of 2.3 ± 0.7 nM. (Ki ± SEM) .PHC-1 was a strong competitor of 125I-Eotaxin binding to CCR3 with half-maximal inhibitory concentration of 78 ± 14 nM (Ki ± SEM). PHC-1 was a strong competitor of 125I-MIP 1-alpha binding to CCR5 with half-maximal inhibitory concentration of 0.04 ± 0.01 nM (Ki ± SEM).

The activity of PHC-1 was tested on CCR1 and CCR3. PHC-lwas more active than RANTES on CCR1 (EC₅₀± SEM: 2.8 ± 0.8 nM compared to 6.3 ± 1.1 nM) and a reasonably good agonist for CCR3 (EC₅₀: 78 ± 14 nM) ), while it was inactive on CCR2, CCR4, CCR6, CCR7, CCR8, CCR9, CXCR1 and CX3CR1.

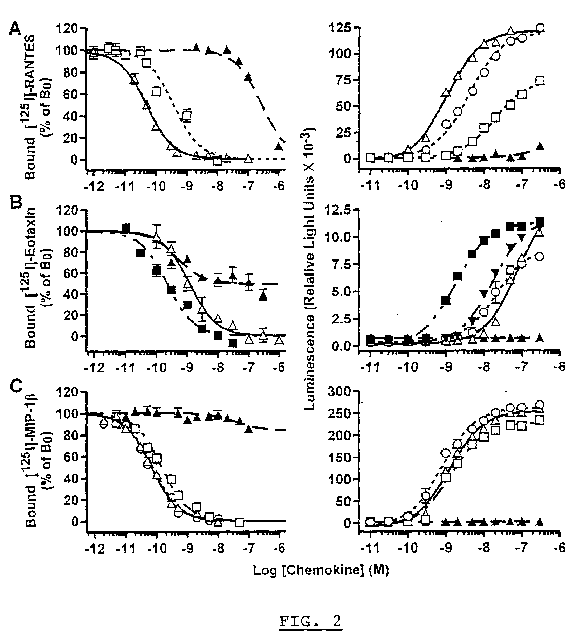

The figure 2 represents the compound PHC-1, full size HCC-1 and reference chemokines tested on CCR1, CCR3 and CCR5. Competition binding assays were performed with [¹²⁵I]-RANTES for CCR1, [¹²⁵I]-Eotaxin for CCR3 and [¹²⁵I]-MIP-1 for CCR5. PHC-1, HCC1, MIP-1, RANTES, Eotaxin and MCP-4 were used as competitors.

The results were normalized for binding in the absence of competitor (100%) and non-specific binding (0%). Functional aequorin-based assays (right panels) were run using CHO-K1 cells expressing the chemokine receptor, apoaequorin and G.

### Example 5: Biological activity of PHC-1 on natural cell populations for its calcium mobilization and chemotactic properties

The migration of cells was assessed by a 48-well microchemotaxis chamber technique (Neuroprobe, Gaithersburg MD). The lower compartment of the chamber was loaded with aliquots of 0.1% BSA medium or of each of the different chemokines concentrations (diluted in 0.1% BSA medium). The upper compartment of the chamber was loaded with a 55-*µ*l cell suspension (5.10⁵ cells/ml in BSA medium) of cells which were previously isolated and washed three times in BSA medium. The two compartments were separated by a polycarbonate PVPF filter, 5*µ*m or 8*µ* m pore size according to the type of cell tested, coated with 20 *µ*g/ml human collagen type IV for 2 h at 37°C. The chamber was incubated for 30 to 180 minutes at 37°C in humidified air with 5% CO₂. At the end of the incubation period, the filter was removed, fixed, and stained with a Diff-Quik kit.

For each chemokine concentration tested, cells migrating through to the underside of the filter were counted in three high power fields ( 500) by light microscopy (after coding the samples) in triplicate. Since the results of several experiments were combined in order to evaluate the migratory response, and since variation in potency of migration were observed between different experiments, the response to each of the chemokine concentrations is shown as a chemotaxis index. The chemotaxis index in each experiment was evaluated by calculating the following ratio: chemotaxis index = the mean of the number of cells migrating to a specific chemokine concentration/the mean of the number of cells migrating to BSA medium (= 0 ng/ml chemokine).

The data are presented as the mean and standard errors (S.E.) of chemotaxis indices of 3-4 experiments. The baseline level of the number of transfected cells migrating was in a similar range for all the cells tested (1-5), with a mean of 6.7, ranging from 2-18 cells per high power field. Due to the large size of the cells and to limitations at the size of the high power field, the maximal response was limited to 70-80 cells per high power field. The p values of migration in response to each of the chemokine concentrations in comparison to migration in response to BSA medium were calculated based on the actual numbers of migrating cells using Student's test.

For intracellular calcium measurements, the cells were loaded for 30 min at room temperature with Fura-2AM. Calcium transients were monitored by a LS 50B spectrofluorimeter (Perkin Elmer) as described in G. Grynkiewicz, M. Poenie, R.Y. Tsien, *J. Biol. Chem.* 260, 3440 (1985).

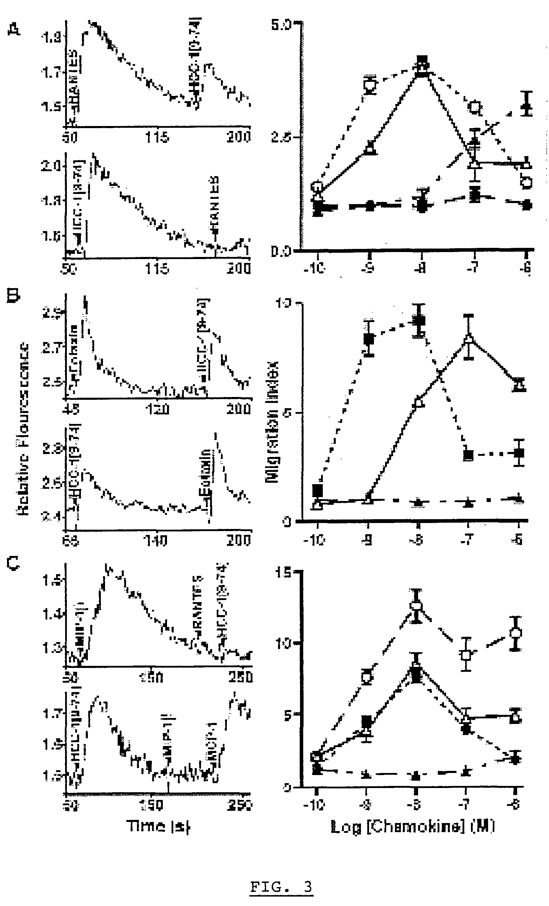

In monocytes, PHC-1 induced robust calcium fluxes, comparable to those evoked by RANTES (Fig. 3A). 50 nM PHC-1 totally desensitized these cells to further stimulation by the same ligand (data not shown), and abrogated further response to 50 nM RANTES as well, while PHC-1 remained capable of mobilizing calcium at reduced levels after a first stimulation by RANTES (Fig. 3A). Prior stimulation by full size HCC-1, MIP-1β or higher RANTES concentrations could not completely abolish the response to PHC-1. Similar results were obtained with the monocytic cell line THP-1. These results suggest that CCR1 and CCR5 mediate part of the functional response of monocytes to PHC-1. In chemotaxis assays with monocytes, PHC-1 was as potent (maximal migration observed at 10 nM) and efficient as RANTES. It was 100-fold more potent than full size HCC-1, which appeared as a weak but efficient chemoattractant (C. L. Tsou et al. (1998) *J. Exp*. *Med.* 188, 603). PHC-1 mobilized calcium less efficiently than eotaxin in eosinophils (Fig. 3B). The eotaxin response was slightly reduced following prior exposure to PHC-1, while the activity of PHC-1 was unaltered following eotaxin stimulation (Fig. 3B) and strongly inhibited after MIP-1α stimulation. These results substantiate the role of PHC-1 as a weak agonist of CCR3, and support the involvement of CCR1 in the functional response of eosinophils to PHC-1. In chemotaxis assays, PHC-1 was less potent but almost as efficacious as eotaxin on eosinophils from most donors. For one of the donors however, HCC-1 was weakly chemotactic at high concentrations only, which was attributed to the low CCR1 expression observed on eosinophils from some individuals (I. Sabroe *et al.* (1999) *J. Immunol.* 162, 2946). PHC-1 but not full size HCC-1 mobilized calcium in interleukin-2 conditioned lymphoblasts. Complete cross-desensitization was observed between the responses to PHC-1 and MIP-1β, indicating that CCR5 is the principal receptor used by PHC-1 in these cells. Migration of lymphoblasts was stimulated by PHC-1 and MIP-1α but not by full size HCC-1 (Fig. 3C). PHC-1 also induced a small calcium flux in neutrophils and desensitization of this response by RANTES but not MIP-1β involved CCR1. No chemotaxis of neutrophils was observed with full length HCC-1 or PHC-1.

As shown in the figure 3, monocytes, eosinophils and IL-2 stimulated lymphoblasts were tested for their functional response to PHC-1, full-size HCC-1 and reference chemokines. Stimulation of calcium mobilization and cross-desensitization experiments of figure 3 were performed with 50 nM chemokine concentrations. The cells were loaded with Fura-2AM and the intracellular calcium concentration ([Ca⁺⁺]ᵢ) was monitored by ratiometric fluorescence measurement (R_{340/380}). Chemotaxis assays were performed in 48-well chambers using polycarbonate filter membranes. Cell migration in response to PHC-1, HCC-1, RANTES, Eotaxin or MIP-1 is reported as a migration index.

### Example 6: Inhibition of HIV-1 entry/replication in human cells by PHC-1

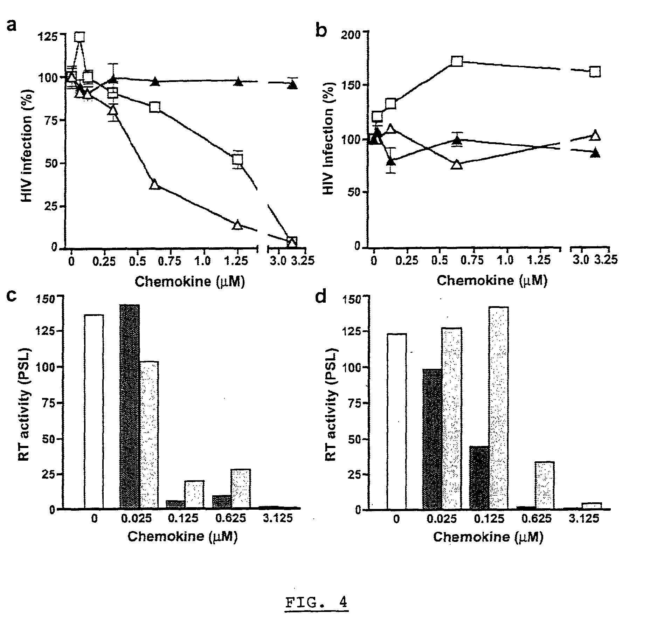

The processed chemokine PHC-1 is a potent inhibitor of HIV replication. The peptide bound to cofactors of HIV-1 entry and efficiently blocked viral replication in cell culture. The anti-viral activity of PHC-1 was investigated in an infection inhibition assay. P4-CCR5 cells (NIH AIDS Research and Reference Reagent program) (P. Charneau *et al.* (1994) *J. Mol. Biol.* 241, 651) were grown in DMEM, supplemented with 10% FCS and 1 *µ*g/ml puromycin. Cells were seeded in flat-bottom 96-well dishes, cultured overnight, and incubated with the chemokines for 2 hours prior to infection with virus containing 20 ng p24 antigen in a total volume of 50 *µ*l medium. After overnight incubation, cells were washed twice and cultivated in fresh DMEM without chemokines. Three days after infection, the cells were lysed and β-galactosidase activity was measured. PBMC were isolated using lymphocyte separation medium (Organon Teknika Corporation). Cells were cultured in RPMI 1640 medium with 20% FCS and 50 U/ml IL-2, and virus production was measured by a reverse transcriptase assay as described (B.J. Potts, *in Techniques in HIV Research,* A. Aldovini, B.D. Walker, Eds. (Stockton, New York, 1990), pp 103-106). Both HCC-1[9-74] and RANTES inhibited infection by the macrophage-tropic strain YU2 (Fig. 4a). RANTES reduced YU2 infection of P4-CCR5 cells by more than 95% at 3.2 *µ*M and by 50% at 1.3 *µ*M. HCC-1 [9-74] was slightly more efficient, blocking YU2 infection by 50% at 0.5 *µ*M. Similar results were obtained using the macrophage-tropic strain JR-CSF. No inhibitory effect on YU2 infection was observed with full-length HCC-1. None of the three chemokines inhibited the T-tropic strain NL4-3 (Fig. 4b). In agreement with previously published results⁸, RANTES enhanced infectivity of NL4-3 by about 50% at concentrations over 0.6 *µ*M. In contrast, no enhancing effect on infectivity was observed with HCC-1[9-74] (Fig. 4b). We also tested whether HCC-1[9-74] could inhibit HIV-1 replication in human peripheral blood mononuclear cells (PBMC). As shown in Fig. 4c & d, replication of the macrophage-tropic strain JR-CSF was blocked significantly by HCC-1 [9-74] and RANTES at concentrations of 125 nM, while concentrations of 625 nM were necessary for the YU2 strain. No inhibition of NL4-3 replication was observed, and HCC-1 was ineffective for all strains.

As shown in the figure 4, cells expressing both CCR5 and CXCR4 co-receptors were infected with the YU2 strain which uses CCR5, and the NL4-3 strain, which uses CXCR4, in the presence of HCC-1, PHC-1 or RANTES. The data represent the mean and s.e.m. for points performed in triplicate. C, D. Human PBMC were infected with YU2 and JR-CSF in the presence of HCC-1, PHC-1 or RANTES. The data represent the reverse transcriptase activity measured in culture cell supernatants harvested 14 days after infection. Results are expressed as photo-stimulated-luminescence (PSL) units.

### Example 7: Generation of biologically active PHC-1 by tryptic digestion of human chemokine HCC-1, amino acid residues 1-74

The amino acid sequence of both newly identified biologically active peptides corresponds to the C-terminal sequence of the human chemokine HCC-1, which originally comprises 74 amino acid residues and was formerly described to occur in nanomolar amounts in human plasma (Schulz-Knappe et al. 1996, Journ. Exp. Med. 183, 295-299). Since the 74 amino residue comprising chemokine HCC-1 with the molecular mass of 8673 Da was completely inactive on CCR5 receptor activation and binding and showed only marginal binding to the CCR1 receptor (Ki 100 nM), we used the 74 amino residue comprising human chemokine HCC-1 as a educt in a digestion with the enzyme trypsin to obtain sufficient amounts of the 65 residue peptide PHC-1 for biological testing. After one hour of incubation (enzyme/substrate-ratio (w/w) 1/100) the 74 residue peptide was in part cleaved into smaller fragments. As one cleavage product also the newly identified biologically active peptide PHC-1, comprising residues 9-74 of the HCC-1 precursor sequence, with the molecular mass of 7795 Da was obtained. The peptide of this invention was further purified to homogeneity (purity > 99%) by two steps of analytical reverse phase chromatography. The peptide PHC-1 obtained from the tryptic digestion of biologically inactive HCC-1, 1-74 was tested in the biological assays as described above and showed full biologically activity (Fig. 2). In contrary, the peptides HCC-1, residues 1-74 and HCC-1, residues 6-74 showed no effect in the assays described above.

### Example 8 : identification of the protease that cleaves full size HCC-1 into PHC-1

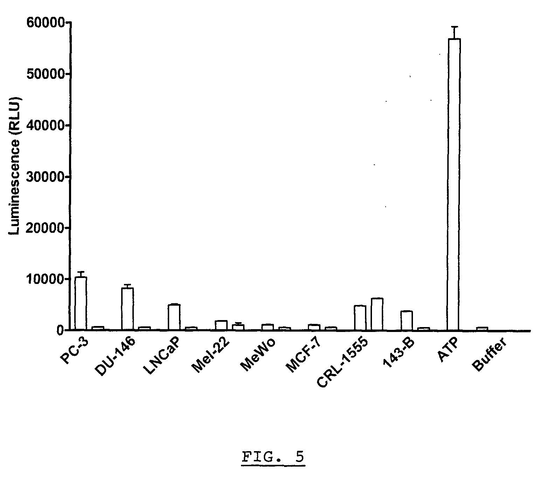

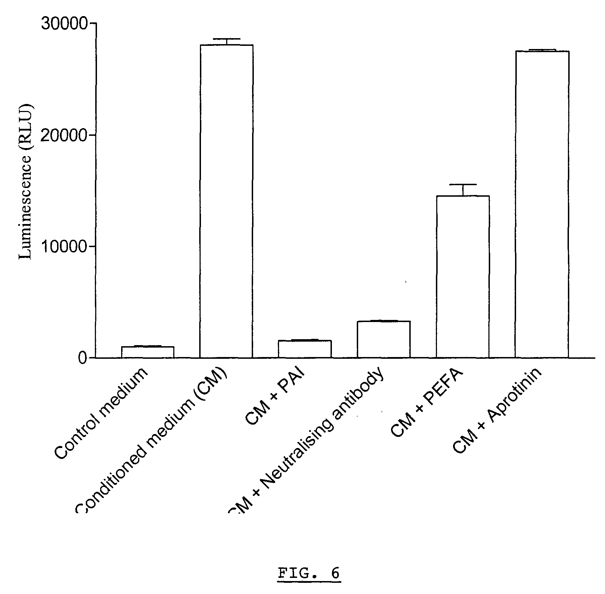

Several human cell lines were screened in order to identify a natural source for the protease generating PHC-1. 1 *µ*M Full size HCC-1 was added to the culture medium of 8 tumoral cell lines: PC-3 (prostatic carcinoma cell line, ATCC # CRL-1435, grown in Nutrient medium Ham's F12 with 10% foetal calf serum, 1 mM sodium pyruvate, 1 mM glutamate, 100 U/ml penicillin and 100 *µ*g/ml streptomycin), Du-146 or 52, (adenocarcinoma cell line (ATCC HTB-81), grown in DMEM medium with 10% foetal calf serum, 1 mM sodium pyruvate, 1 mM glutamate, 100 U/ml penicillin and 100 *µ*g/ml streptomycin), 143-B (osteosarcoma cell line, ATCC # CRL-8303, grown in DMEM medium with 10% foetal calf serum, 1 mM sodium pyruvate, 1 mM glutamate, 100 U/ml penicillin and 100 *µ*g/ml streptomycin), Mel-22 and MeWo (melanoma cell lines, grown in DMEM medium with 10% foetal calf serum, 1 mM sodium pyruvate, 1 mM glutamate, 100 U/ml penicillin and 100 *µ*g/ml streptomycin), MCF-7 (adenocarcinoma cell line, ATCC # HTB-22, grown in RPMI1640 medium with 10% foetal calf serum, 1 mM sodium pyruvate, 1 mM glutamate, 100 U/ml penicillin and 100 *µ*g/ml streptomycin) and CRL-1555 (epidermoid carcninoma cell line, ATCC # A431, grown in RPMI1640 medium with 10% foetal calf serum, 1 mM sodium pyruvate, 1 mM glutamate, 100 U/ml penicillin and 100 *µ*g/ml streptomycin). Following an incubation of 48 hours at 37°C, the culture medium was centrifuged and tested in an aequorin assay using CCR5 expressing cells, as described in example 3. Figure 5 shows that PC-3, DU-146 and 143-B activated full size HCC-1. Non specific response was only obtained with the CRL-1555 culture medium without addition of full size HCC-1. Serum-free conditionned media obtained from these different cell lines were also able to activate full size HCC-1, indicating that the protease generating PHC-1 was soluble. As PC-3 secretes high amount of urokinase, a serine protease, several serine protease inhibitors were tested in PC-3 conditioned medium incubated with 500 nM full size HCC-1, during 6 hours at 37°C. As shown in figure 6, all the proteases inhibitors decreased the activation of full size HCC-1. 3 *µ*g/ml PAI-1, a specific urokinase inhibitor, was more efficient than 4 mM Pefabloc; 2 *µ*g/ml aprotinin was inactive. Furthermore, a specific blocking urokinase antibody (monoclonal neutralizing antibody 394 against human uPA B-chain, American Diagnostica, USA) also inhibited the activation of full size HCC-1. Purified CHOAY urokinase (Sanofi Wintrop) was then incubated at 37°C for 40 min with 500 nM full size HCC-1. The reaction product was purified on reverse phase-HPLC and the fractions were tested in the aequorin assay with CCR5 expressing cells. The active fractions were then analysed by mass spectometry and Edman degradation, identifying PHC-1 and full size HCC-1 as the major compounds in these fractions. As specified above, trypsin generated PHC-1 then degrades PHC-1 in inactive fragments when the incubation time is increased. 50 nM PHC-1 was incubated with 10 or 100 IU purified urokinase. No change of activity was observed after an incubation of 90 min at 37°C, indicating that urokinase do not degrade PHC-1.

## Claims

1. Compound which presents more than 60% homology with SEQ ID NO: 1, with the proviso that said compound does not correspond to the amino acid sequence SEQ ID NO: 3, SEQ ID NO: 4 or their biologically active amidated, acetylated, phosphorylated and/or glycosylated derivatives.

2. Compound according to claim 1, which presents more than 75% homology with SEQ ID NO: 1, with the proviso that said compound does not correspond to the amino acid sequence SEQ ID NO: 3, SEQ ID NO: 4 or their biologically active amidated, acetylised, phosphorylised and/or glycosyled derivatives.

3. Compound according to claim 1 or 2, which presents more than 85% homology with SEQ ID NO: 1, with the proviso that said compound does not correspond to the amino acid sequence SEQ ID NO: 3, SEQ ID NO: 4 or their biologically active amidated, acetylated, phosphorylated and/or glycosylated derivatives.

4. Compound according to any of the preceding claims 1 to 3, **characterised in that** it presents more than 95% homology with SEQ ID NO: 1, with the proviso that said compound does not correspond to the amino acid sequence SEQ ID NO: 3, SEQ ID NO. 4 or their biologically active amidated, acetylated, phosphorylated and/or glycosylated derivatives.

5. Chemokine compound, **characterised in that** it has the amino acid sequence SEQ ID NO: 1, SEQ ID NO: 2 or biologically active fragments or portions thereof.

6. Chemokine compound comprising the amino acid sequence SEQ ID NO: 1, SEQ ID NO: 2 or biologically active fragments or portions thereof, modified by or linked to one or more amide, acetyl, phosphoryl and/or glycosyl groups.

7. Chemokine compound according to any of the preceding claims 1 to 6, binding to a chemokine receptor, preferably one or more receptors selected from the group consisting of CCR-1, CCR-3 and CCR-5 receptors.

8. Polynucleotide encoding the amino acid sequence of the compound according to any of the preceding claims.

9. Antagonist or inhibitor of the compound or the polynucleotide encoding the amino acid sequence of said compound according to any of the preceding claims to a chemokine receptor, preferably to a receptor selected from the group consisting of CCR-1, CCR-3 and CCR-5 chemokine receptors, with the proviso that said antagonist or inhibitor is not a natural known compound.

10. The inhibitor according to the claim 9, **characterised in that** it is a (possibly labelled) antibody or an active hypervariable portion of an antibody.

11. The inhibitor according to the claim 9, **characterised in that** it is an antisense oligonucleotide or a ribozyme having a sequence of at least 15 nucleotides capable of specifically hybridising to a mRNA molecule encoding the compound according to the invention, so as to prevent the translation of said mRNA molecule or an antisense oligonucleotide capable of specifically hybridising to a DNA molecule encoding the compound according to the invention, said antisense oligonucleotide comprising possibly chemical analogues of nucleotides.

12. Vector comprising the polynucleotide according to the claim 8.

13. Cell transformed by the vector according to the claim 12.

14. A method for the screening (of molecules which are not known to be agonists, antagonists or inhibitors of the compound according to any of the preceding claims 1 to 7, to a chemokine receptor, especially to a CCR-5 chemokine receptor, and comprising the steps of :
- contacting a cell or cell extract from the cells transfected with a vector expressing a polynucleotide encoding said receptor,
- possibly isolating a membrane fraction from the cell extract or the complete cell with the compound according to the invention under conditions permitting binding of said compound to said receptor, possibly by the activation of a functional response, and
- detecting the presence of any such compound by means of a bio-assay (such as a modification, the production of a second messenger or an increasing in the receptor activity) in the presence of the molecule working as an agonist, antagonist or inhibitor to the compound, thereby (possibly recovering and) determining whether said molecule is able to work as an agonist, antagonist or inhibitor of the compound according to the invention to its receptor.

15. The method according to the claim 13, **characterised in that** the messenger assay comprises the measurement of intracellular CAMP, intracellular inositol phosphate, intracellular diacylglycerol concentration, arachidonic acid concentration, MAP kinase or tyrosine kinase pathways or intracellular calcium mobilisation.

16. An antagonist or inhibitor of the compound according to any of the preceding claims 1 to 7, identified and possibly recovered by the method according to any of the preceding claims 14 or 15.

17. A molecule responsible for the regeneration of the compound according to any one of the claims 1 to 7, preferably a protease able to cleave the compound having the amino acid sequence SEQ ID NO: 3 or SEQ ID NO: 4 into amino acid sequences SEQ ID NO: 1 or SEQ ID NO: 2.

18. The molecule according to the claim 17, being a (prefereably) recombinant urokinase or streptokinase.

19. An inhibitor or activator of the molecule according to the claim 17 or 18, for improving the generation of the compound according to any one of the claims 1 to 7, *in vitro* or *in vivo*.

20. Pharmaceutical composition comprising an adequate pharmaceutical carrier and the compound according to the claims 1 to 7, the polynucleotide according to claim 8, the antagonist or inhibitor according to the claim 9 or 16, the molecule according to the claims 17 or 18 and/or an inhibitor or activator of said molecule according to the claim 19.

21. Use of the pharmaceutical composition according to the claim 20 for the manufacture of a medicament in the prevention and/or the treatment of a disease induced by viral infections, in particular diseases induced by immunodeficiency viruses 1 and/or 2 (HIV-1 and/or HIV-2) or viruses selected from the group consisting of herpes simplex virus, varicella zoster virus, hepatitis-A viruses, hepatitis-B viruses, zytomegalo virus, influenza virus, polio virus, rhino virus, measles virus, German measles virus, rabies virus, Rous sarcoma virus, Epstein-Barr virus, Pox virus, a bacterial agent or a protosea.

22. Use of the pharmaceutical composition according to the claim 20 for the manufacture of a medicament in the prevention and/or the treatment of a disease selected from the group consisting of inflammation (including rheumatoid arthritis), cancers (including Hodgkins lymphomas), restenosis, atherosclerosis, allergies (including asthma), psoriasis, chronic contact dermatitis, inflammatory bowel disease, multiple sclerosis, stroke, sarcoidosis, organ transplant rejection, infection induced by pathogeneous agent including viral infections by HIV-1 and/or HIV-2 (AIDS).

23. Diagnostic kit or device comprising the compound according to claim 1 to 7, the polynucleotide according to claim 8, the agonist, antagonist or inhibitor according to the claim 9 or 16, the molecule according to the claim 17 or 18 and/or an inhibitor or activator of said molecule according to the claim 19.
